# EUROPEAN PATENT APPLICATION

(11) **EP 3 832 379 A1**
(43) Date of publication of application: **09.06.2021**
(21) Application number: 20171807.9
(22) Date of filing: 28.04.2020
(51) Int. Cl.: G02C 11/04, A61N 5/06, G02C 7/10, G02C 7/14, A45C 11/04

(54) **PHOTOTHERAPY GLASSES AND GLASSES CASE**

(30) Priority: 05.12.2019 CN 201911235702
(71) Applicant: Beijing Xiaomi Mobile Software Co., Ltd., Beijing 100085 (CN); Lumos Health Inc., Waterloo, Ontario N2L 4R7 (CA)
(72) Inventor: WEN, Chengyu, Beijing, Beijing 100085 (CN); SUN, Fei, Beijing, 100085 (CN)
(74) Representative: dompatent von Kreisler Selting Werner - Partnerschaft von Patent- und Rechtsanwälten mbB

(57) **Abstract**

Phototherapy glasses (100) and a glasses case are provided. The phototherapy glasses (100) include a front frame (1), a first temple (2), and a second temple (3), the first temple (2) and the second temple (3) being hinged to either side of the front frame (1) respectively, so that the first temple (2) and the second temple (3) can be folded and unfolded relative to the front frame (1); a light source component (4) disposed on the first temple (2) and/or the second temple (3); a lens (5) disposed on the front frame (1); and a reflecting layer (6), the reflecting layer (6) being formed on the lens (5) and configured to reflect light from the light source component (4), so that the light is reflected to the eyes of a user.

## Description

### TECHNICAL FIELD

The disclosure relates to the technical field of terminals, and more particularly, to phototherapy glasses and a glasses case.

### BACKGROUND

Visible phototherapy can use artificial light to simulate sunlight, which helps to treat various mental conditions and other symptoms related to the human biological clock, such as seasonal affective disorders, depression, delayed sleep disorders, and insomnia.

In a related art, a phototherapy lamp may be provided. A user needs to sit near the phototherapy lamp and enable light emitted by the phototherapy lamp to reach the eyes, so as to achieve the purpose of phototherapy. However, due to the disadvantages of heavy weight of the phototherapy lamp and tedious position movement, the user cannot move freely during the therapy.

In another related art, a wearable phototherapy product may be provided. A user can perform some daily activities, such as sweeping a floor and reading a book, while wearing the wearable phototherapy product to achieve the purpose of phototherapy. However, current wearable phototherapy products generally have special or strange product forms, which affect the aesthetics and convenience of patients when they are worn.

### SUMMARY

The disclosure provides phototherapy glasses and a glasses case to solve the defects in the related art.

According to a first aspect of embodiments of the disclosure, phototherapy glasses are provided. The phototherapy glasses includes:
a front frame, a first temple and a second temple, the first temple and the second temple being hinged to either side of the front frame respectively, so that the first temple and the second temple are able to be folded and unfolded relative to the front frame;
a light source component disposed on the first temple and/or the second temple;
a lens disposed on the front frame; and
a reflecting layer, the reflecting layer being formed on the lens and configured to reflect light from the light source component, so that the light is reflected to the eyes of a user.

The technical solutions provided by the embodiments of the disclosure may include the following beneficial effects.

It can be known from the above embodiments that the phototherapy glasses disclosed in the disclosure can be stored and worn through the folding and unfolding of the first temple and the second temple relative to the front frame. The structure can be simplified as compared to the phototherapy glasses in the related art. Further, the light source component is disposed on the first temple or the second temple and cooperates with the reflection effect of the reflecting layer to simplify the appearance structure of the phototherapy glasses while achieving the phototherapy effect.

Optionally, the reflecting layer may be disposed on a side of the lens facing the eyes of the user.

Optionally, the light source component may include a light source and a plano-convex lens, the plano-convex lens may include a light incident surface and a light exit surface, and light emitted by the light source may enter the light incident surface of the plano-convex lens and exit from the light exit surface perpendicularly.

Optionally, the light source may be located at a focal point of the lens.

Optionally, an angle formed by the light exiting from the light exit surface and the lens may be between 25° and 55°.

Optionally, the reflecting layer may be a non-metallic dielectric film layer.

Optionally, the reflecting layer may include a dichroic coating or a dielectric coating.

Optionally, the front frame may include a first support frame, a second support frame, and a connecting member connecting the first support frame and the second support frame. The first support frame may be hinged to the first temple, and the second support frame may be hinged to the second temple.

The lens may include a first lens disposed on the first support frame and a second lens disposed on the second support frame.

The first temple and the second temple may be provided with the light source component respectively, and the first lens and the second lens may be provided with the reflecting layer respectively.

Optionally, the light source component may include a first light source located in the first temple and a second light source located in the second temple.

The phototherapy glasses may further include a light source control circuit and a battery. The battery may supply power to the first light source and the second light source through the light source control circuit.

Optionally, the phototherapy glasses may further include a charging module. The charging module may be electrically connected to the battery and configured to charge the battery.

Optionally, the phototherapy glasses may further include a color changing layer formed on the lens.

Optionally, the light source may include an LED light source.

Optionally, the first support frame and the second support frame may include an opening separately, so that the lens is able to be fitted into the openings and fixed.

According to a second aspect of the embodiments of the disclosure, a glasses case is provided. The glasses case cooperates with the phototherapy glasses according to any one of the above embodiments. The glasses case includes a charging component. The charging component cooperates with the phototherapy glasses to charge the phototherapy glasses through the glasses case.

Optionally, the charging component may include a charging interface and/or a wireless charging coil.

It is to be understood that the above general descriptions and detailed descriptions below are only exemplary and explanatory and not intended to limit the disclosure.

### BRIEF DESCRIPTION OF THE DRAWINGS

The accompanying drawings, which are incorporated in and constitute a part of this specification, illustrate embodiments consistent with the disclosure and, together with the specification, serve to explain the principles of the disclosure.
Fig. 1 is a schematic structural diagram illustrating phototherapy glasses according to an exemplary embodiment.
Fig. 2 is a schematic wearing diagram illustrating phototherapy glasses according to an exemplary embodiment.
Fig. 3 is a schematic structural diagram illustrating a light source component according to an exemplary embodiment.
Fig. 4 is a structural block diagram illustrating phototherapy glasses according to an exemplary embodiment.

### DETAILED DESCRIPTION

Reference will now be made in detail to exemplary embodiments, examples of which are illustrated in the accompanying drawings. The following description refers to the accompanying drawings in which the same reference numeral in different drawings represents the same or similar elements unless otherwise specified. The implementations set forth in the following description of exemplary embodiments do not represent all implementations consistent with the disclosure. Instead, they are merely examples of apparatuses and methods consistent with aspects related to the disclosure as recited in the appended claims.

The terms used in the disclosure are for the purpose of describing particular embodiments only, and are not intended to limit the disclosure. "A/an", "the" and "said" in a singular form in the disclosure and the appended claims are also intended to include a plural form, unless other meanings are clearly specified throughout the disclosure. It should also be understood that term "and/or" used in the disclosure refers to and includes any or all possible combinations of one or more associated items that are listed.

It should be understood that although the terms first, second, third, etc. may be used in the disclosure to describe various information, such information should not be limited to these terms. These terms are only used to distinguish the same type of information from each other. For example, without departing from the scope of the disclosure, first information may also be called second information and, similarly, second information may also be called first information. For example, term "if" used here may be explained as "while" or "when" or "responsive to determining", which depends on the context.

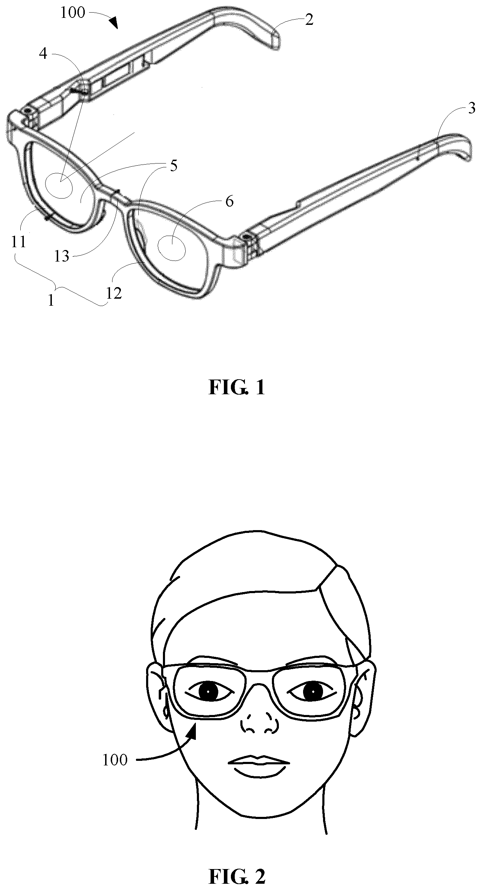

Fig. 1 is a schematic structural diagram illustrating phototherapy glasses 100 according to an exemplary embodiment. As shown in FIG. 1, the phototherapy glasses 100 may include a front frame 1, a first temple 2 and a second temple 3. The first temple 2 and the second temple 3 are hinged to either side of the front frame 1 respectively. Therefore, the first temple 2 and the second temple 3 can be folded in a direction close to the front frame 1, and the phototherapy glasses 100 are switched to a folded state and are convenient to carry. Both the first temple 2 and the second temple 3 can be unfolded in a direction away from the front frame 1, and the phototherapy glasses 100 are switched to an unfolded state. Therefore, the phototherapy glasses 100 may be worn on the face of a user as shown in FIG. 2, and phototherapy light may be emitted into the eyes of the user to achieve the phototherapy effect.

Further, the phototherapy glasses 100 may further include a light source component 4, a lens 5 and a reflecting layer 6. The light source component 4 may be disposed on at least one of the first temple 2 and the second temple 3. The lens 5 may be disposed on the front frame 1. The reflecting layer 6 may be formed on the lens 5. The reflecting layer 6 may be configured to reflect light from the light source component 4, so that the light from the light source component 4 can be reflected to the eyes of the user to achieve the phototherapy effect. The phototherapy glasses 100 may further include a color changing layer formed on the lens 5. The color changing layer may change color according to the intensity of irradiated ultraviolet light. For example, when the ultraviolet light is strong, the color of the color changing layer is darkened, and light entering the eyes of the user is weakened. When the ultraviolet light is weak, the color of the color changing layer is lighter, which is beneficial to light transmission and makes the eyes of the user more adaptable to current environmental conditions.

It can be known from the above embodiments that the phototherapy glasses disclosed in the disclosure can be stored and worn through the folding and unfolding of the first temple and the second temple relative to the front frame. The structure can be simplified as compared to the phototherapy glasses in the related art. Further, the light source component 4 is disposed on the first temple 2 or the second temple 3 and cooperates with the reflection effect of the reflecting layer 6 to simplify the appearance structure of the phototherapy glasses while achieving the phototherapy effect.

In this embodiment, the reflecting layer 6 may be disposed on a side of the lens 5 facing the eyes of the user, thereby avoiding the light from the light source component 4 from being weakened or refracted during the process of light transmission through the lens, which is beneficial to ensure the phototherapy effect. Further, the reflecting layer 6 may allow light of a fixed frequency to be reflected, and light of a frequency other than the fixed frequency may pass through the reflecting layer, so that the user can see the outside world with the phototherapy glasses while ensuring the phototherapy effect.

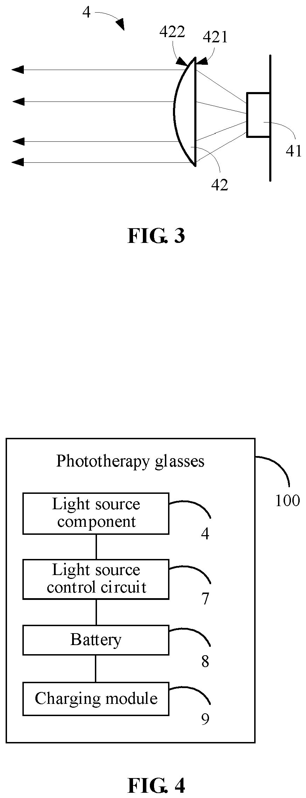

In each of the above embodiments, as shown in FIG. 3, the light source component 4 may include a light source 41 and a plano-convex lens 42. The plano-convex lens 42 includes a light incident surface 421 and a light exit surface 422. Light emitted by the light source 41 may enter the light incident surface 421 of the plano-convex lens 42 and may exit from the light exit surface perpendicularly. Based on this, it is possible to convert the scattered light emitted by the light source 41 into mutually parallel light, and further into linear light that can be emitted to the reflecting layer 6, thereby reducing the amount of light loss.

In this embodiment, the light source 41 may be disposed at a focal point of the lens 5, and an angle formed by parallel light exiting from the plano-convex lens 42 and the lens 5 may be between 25° and 55°. Therefore, the light from the light source 41 may be emitted to the reflecting layer 6 obliquely. By adjusting the position of the reflecting layer 6 and an incident angle of the light, it is beneficial to make the light from the light source enter the eyes of the user perpendicularly, thereby improving the visual effect and the phototherapy effect. The light source 41 may include an LED light source, such as blue LED light of 465nm, blue light with the illumination intensity of 100lux, green light with the illumination intensity of 350lux or white light with the illumination intensity of 1000-5000lux. It may be specifically designed according to therapy requirements and will not be limited in the disclosure.

Based on the technical solution of the disclosure, the reflecting layer 6 may include a non-metallic dielectric film layer, which is beneficial to realize the connection between the reflecting layer 6 and the lens 5. For example, the reflecting layer 6 may include a dichroic coating or a dielectric coating. When the reflecting layer 6 is a dielectric coating, for example, a film layer which may include 25-30 layers, and the thickness of the reflecting layer 6 is, for example, 2um±1um, the reflecting layer may be specifically designed as required and will not be limited in the disclosure.

In the technical solution of the disclosure, as shown in FIG. 1, the front frame 1 may include a first support frame 11, a second support frame 12, and a connecting member 1 connecting the first support frame 11 and the second support frame 12. The first support frame 11 may be hinged to the first temple 2, and the second support frame 12 may be hinged to the second temple 3. Moreover, the first support frame 11 and the second support frame 12 may include an opening separately, so that the lens 5 may be fitted into the openings and fixed. In one example, the lens 5 may include a first lens disposed in the first support frame 11 and a second lens disposed in the second support frame 12.

Therefore, in order to equalize the light intensity felt by the two eyes of the user, the first lens and the second lens may be provided with the reflecting layer 6 respectively, and the first temple 2 and the second temple 3 may be provided with the light source component 4 respectively. The switching states of the light source components 4 disposed on the first temple 2 and the second temple 3 may be synchronized to equalize light reflected into different eyes of the user.

In each of the above embodiments, as shown in FIG. 4, the light source 41 may include a first light source located on the first temple 2 and a second light source located on the second temple 3. Further, the phototherapy glasses 100 may further include a light source control circuit 7 and a battery 8. In one embodiment, the battery 8 may be located in the first temple 2 or the second temple 3, one end of the light source control circuit 7 may be electrically connected to the battery 8, and the other end may be connected to the first light source and the second light source, so that the first light source and the second light source are powered by the battery 8. In another embodiment, the battery 8 may include a first battery in the first temple 2 and a second battery in the second temple 3. Similarly, the light source control circuit 7 may include a first circuit located in the first temple 2 and a second circuit located in the second temple 3. The first circuit is connected to the first light source and the first battery, and the second circuit is connected to the second light source and the second battery, so that the first battery supplies power to the first light source, and the second battery supplies power to the second light source.

Further, as shown in FIG. 4, the phototherapy glasses 100 may further include a charging module 9. The charging module 9 is electrically connected to the battery 8, so that the battery can be charged when the charging module 9 is connected to an external power source. The external power source may be an external power socket or a power bank or another discharge terminal.

Based on the phototherapy glasses 100 in each of the above embodiments, in order to protect the phototherapy glasses 100 during non-therapy periods, the disclosure also provides a glasses case (not shown in the figure). The glasses case may cooperate with the phototherapy glasses 100 in each of the above embodiments. Furthermore, the glasses case may further include a charging component. The charging component may cooperate with the phototherapy glasses 100, so that the phototherapy glasses 100 can be charged through the glasses case, and a situation of insufficient power when a user starts the phototherapy glasses 100 can be avoided. In one example, the charging component may include a physical charging interface. The physical charging interface is electrically connected to the charging module 9 of the phototherapy glasses 100 for charging. The physical charging interface may include a USB-C type interface. In another example, the charging component may also charge the phototherapy glasses by wireless charging. For example, the charging component includes an electromagnetic induction type wireless charging coil and forms a charging area inside the glasses case. When the phototherapy glasses 100 are placed in the glasses case and the charging module 9 of the phototherapy glasses 100 is aligned with the charging area in the glasses case, charging can be realized, and the use of a data cable is avoided, which is convenient and quick. Alternatively, the phototherapy glasses may also be wirelessly charged by being attached to the glasses case in a contact manner.

In one embodiment, the glasses case itself may also be charged in a wired or wireless manner, which is not repeated here.

In one embodiment, the glasses case may further include an indicator light for displaying a charging state of the phototherapy glasses. Alternatively, the glasses case may further include a display screen for displaying the current power of the phototherapy glasses.

Other embodiments of the disclosure will be apparent to those skilled in the art from consideration of the specification and practice of the disclosure disclosed here. The disclosure is intended to cover any variations, uses, or adaptations of the disclosure following the general principles thereof and including common knowledge or conventional technical means in the art not disclosed in the disclosure. It is intended that the specification and examples be considered as exemplary only, with a true scope of the disclosure being indicated by the following claims.

It will be appreciated that the disclosure is not limited to the exact construction that has been described above and illustrated in the accompanying drawings, and that various modifications and changes can be made without departing from the scope thereof. It is intended that the scope of the disclosure is only limited by the appended claims.

## Claims

1. Phototherapy glasses (100), **characterized in that** the phototherapy glasses (100) comprises:
a front frame (1), a first temple (2) and a second temple (3), the first temple (2) and the second temple (3) being hinged to either side of the front frame (1) respectively, so that the first temple (2) and the second temple (3) are able to be folded and unfolded relative to the front frame (1);
a light source component (4) disposed on the first temple (2) and/or the second temple (3);
a lens (5) disposed on the front frame (1); and
a reflecting layer (6), the reflecting layer (6) being formed on the lens (5) and configured to reflect light from the light source component (4), so that the light is reflected to the eyes of a user.

2. The phototherapy glasses (100) according to claim 1, wherein the reflecting layer (6) is disposed on a side of the lens (5) facing the eyes of the user.

3. The phototherapy glasses (100) according to claim 1 or 2, wherein the light source component (4) comprises a light source (41) and a plano-convex lens (42), the plano-convex lens (42) comprises a light incident surface (421) and a light exit surface (422), and light emitted by the light source (41) enters the light incident surface (421) of the plano-convex lens (42) and exits from the light exit surface (422) perpendicularly.

4. The phototherapy glasses (100) according to any of claims 1 to 3, wherein the light source (41) is located at a focal point of the lens (5).

5. The phototherapy glasses (100) according to any of claims 1 to 4, wherein an angle formed by the light exiting from the light exit surface (422) and the lens (5) is between 25° and 55°.

6. The phototherapy glasses (100) according to any of claims 1 to 5, wherein the reflecting layer (6) is a non-metallic dielectric film layer.

7. The phototherapy glasses (100) according to any of claims 1 to 6, wherein the reflecting layer (6) comprises a dichroic coating or a dielectric coating.

8. The phototherapy glasses (100) according to any of claims 1 to 7, wherein the front frame (1) comprises a first support frame (11), a second support frame (12), and a connecting member connecting the first support frame (11) and the second support frame (12), the first support frame (11) being hinged to the first temple (2), and the second support frame (12) being hinged to the second temple (3);
the lens (5) comprises a first lens disposed on the first support frame (11) and a second lens disposed on the second support frame (12); and
the first temple (2) and the second temple (3) are provided with the light source component (4) respectively, and the first lens and the second lens are provided with the reflecting layer (6) respectively.

9. The phototherapy glasses (100) according to any of claims 1 to 8, wherein the light source component (4) comprises a first light source located in the first temple (2) and a second light source located in the second temple (3); and
the phototherapy glasses (100) further comprise a light source control circuit (7) and a battery (8), the battery (8) supplying power to the first light source and the second light source through the light source control circuit (7).

10. The phototherapy glasses (100) according to claim 9, further comprising a charging module (9) electrically connected to the battery (8) and configured to charge the battery (8).

11. The phototherapy glasses (100) according to any of claims 1 to 10, wherein the phototherapy glasses (100) further comprise a color changing layer formed on the lens (5).

12. The phototherapy glasses (100) according to any of claims 1 to 11, wherein the light source (41) comprises an LED light source.

13. The phototherapy glasses (100) according to any of claims 1 to 12, wherein the first support frame (11) and the second support frame (12) comprise an opening separately, so that the lens (5) is able to be fitted into the openings and fixed.

14. A glasses case, **characterized in that** the glasses case cooperates with the phototherapy glasses (100) according to any one of claims 1 to 13, the glasses case comprising a charging component, the charging component cooperating with the phototherapy glasses (100) to charge the phototherapy glasses (100) through the glasses case.

15. The glasses case according to claim 14, wherein the charging component comprises a charging interface and/or a wireless charging coil.
